# EUROPÄISCHE PATENTSCHRIFT

(11) **EP 1 850 834 B1**
(45) Veröffentlichungstag und Bekanntmachung des Hinweises auf die Patenterteilung: **20.08.2014**
(21) Anmeldenummer: 06707853.5
(22) Anmeldetag: 26.01.2006
(51) Int. Cl.: A61K 9/28, A61K 9/20

(54) **FILMTABLETTEN ENTHALTEND IBUPROFEN-LYSINAT**
FILM-COATED TABLETS CONTAINING IBUPROFEN LYSINATE
COMPRIMES COUVERTS D'UN FILM, CONTENANT DU LYSINATE D'IBUPROFENE

(30) Priorität: 09.02.2005 EP 05002682
(43) Veröffentlichungstag der Anmeldung: 07.11.2007
(73) Patentinhaber: Boehringer Ingelheim International GmbH, 55218 Ingelheim am Rhein (DE); Boehringer Ingelheim Pharma GmbH & Co. KG, 55218 Ingelheim am Rhein (DE); Dr. R. Pfleger Gmbh, 96045 Bamberg (DE)
(72) Erfinder: ANSCHÜTZ, Sergej, 55452 Rümmelsheim (DE); SCHNEIDER, Roland, 65388 Schlangenbad (DE); JUNG, Gerd, 96149 Breitengüssbach (DE); SCHAUPP, Albert, 96129 Strullendorf (DE)
(74) Vertreter: Simon, Elke Anna Maria
(86) Internationale Anmeldenummer: PCT/EP2006/050466
(87) Internationale Veröffentlichungsnummer: WO 2006/084793

(56) Entgegenhaltungen:
- WO-A-97/30699

## Beschreibung

### HINTERGRUND DER ERFINDUNG

### 1. TECHNISCHES GEBIET

Die Erfindung betrifft Filmtabletten enthaltend Ibuprofen-Lysinat mit verbesserter Stabilität, Bioverfügbarkeit und höherer Akzeptanz (Compliance) bei Patienten, sowie die Verwendung dieser Filmtabletten zur Herstellung eines Arzneimittels zur Behandlung von akuten und/oder chronischen Schmerzen.

### 2. STAND DER TECHNIK

Die britische Patentanmeldung GB 1 471 910 beschreibt Ibuprofen-Lysinat als wasserlösliche Form von Ibuprofen mit verbesserter therapeutischer Wirkung. Die europäische Patentanmeldung EP 0 172 014 schlägt Ibuprofen Formulierungen mit einem hohen Gehalt an Ibuprofen vor, die als Trägerstoff 1 bis 15 Gew.-% Croscarmellose Natrium aufweisen. In der europäischen Patentanmeldung EP 0 411 952 werden kaubare Tabletten mit schneller Wirkstofffreisetzung beschrieben, worin der Wirkstoff, insbesondere Ibuprofen, in Form von verpressten Körnchen, welche mit Polyvinylpyrrolidon und Natriumlaurylsulfat granuliert wurden, enthalten ist. Die europäische Patentanmeldung EP 0 505 180 schlägt eine Sprengmittel-freie Tablette mit einem Gehalt an Ibuprofen-Lysinat von über 90 Gew.-% vor.

WO 97/30699 offenbart schnell zerfallende Ibuprofen Tabletten.

Das bekannte Präparat Dolormin® ist eine Filmtablette, welche aus Ibuprofen-Lysinat, mikrokristalliner Cellulose, Polyvinylpyrrolidon (Povidon), Magnesiumstearat, Titandioxid, Hypromellose und Hydroxypropylcellulose besteht.

Polyvinylpyrrolidon (Povidon) fungiert dabei als Trockenbindemittel. Diese Substanz ist jedoch leicht hygroskopisch und führt dazu, dass die entsprechenden Tablettenmischungen insbesondere bei höheren Luftfeuchten beim Verpressen zum Kleben neigen, was im Produktionsprozeß zu erheblichen Nachteilen führen kann (z.B. Verkleben der Werkzeuge). Außerdem zeigt Povidon in Abhängigkeit von der Gesamtzusammensetzung des Produktes Wechselwirkungen mit anderen Substanzen, z.B. können Verfärbungen auftreten.

Bei Einsatz von höheren Mengen in Tablettenformlierungen ist bedingt durch die gute Wasserlöslichkeit der Substanz in Abhängigkeit von der Gesamtzusammensetzung ein verzögernder Einfluss auf die Zerfallszeit der Darreichungsform nicht ausgeschlossen.

Der vorliegenden Erfindung lag die Aufgabe zu Grunde eine Ibuprofen-haltige Filmtablette zur Verfügung zu stellen, die eine vergleichbare oder bessere therapeutische Wirksamkeit wie Dolormin® aufweist unter Vermeidung der oben genannten Nachteile.

### KURZE BESCHREIBUNG DER ERFINDUNG

Es wurde nun überraschenderweise gefunden, dass bei der Verwendung einer bestimmten Kombination von Trägerstoffen des Tablettenkerns auf das Trockenbindemittel Povidon verzichtet werden kann und die damit verbundenen Nachteile vermieden werden können, wobei gleichzeitig die günstigen therapeutischen Eigenschaften beibehalten bzw. sogar verbessert werden.

Gegenstand der Erfindung ist somit eine Ibuprofen-haltige Filmtablette, wobei der Tablettenkern aus folgenden Komponenten besteht:
(a) 80,0-85,0 Gew.-% des Lysinats von racemischem (R/S)-Ibuprofen,
(b) 12,5-17,5 Gew.-% Mikrokristalline Cellulose,
(c) 0,1-1,0 Gew.-% hochdisperses SiO₂, und
(d) 0,75-1,5 Gew.-% Magnesium Stearat.

Ein weiterer Gegenstand der Erfindung ist die Verwendung dieser Ibuprofen-haltigen Filmtablette zur Herstellung eines Arzneimittels zur Behandlung von akuten und/oder chronischen Schmerzen.

### DETAILLIERTE BESCHREIBUNG DER ERFINDUNG

Der Begriff Ibuprofen-Lysinat bedeutet vor und nachstehend (±) -2 - (p-Isobutylphenyl) propionsäure-lysin Salz, insbesondere in Form des Monohydrats.

Vorzugsweise besteht der Tablettenkern aus (%-Angaben beziehen sich auf den Tablettenkern):
(a) 83,0-84,0, insbesondere etwa 83,4 Gew.-% des Lysinats von racemischem (R/S)-Ibuprofen,
(b) 14,5-15,5, insbesondere etwa 15,0 Gew.-% mikrokristalline Cellulose,
(c) 0,3-0,4, insbesondere etwa 0,37 Gew.-% hochdisperses SiO₂, und
(d) 1,0-1,4, insbesondere etwa 1,22 Gew.-% Magnesium Stearat.

Vorzugsweise enthält eine einzelne Filmtablette 342, 684 oder 1026 mg Ibuprofen-Lysinat entsprechend 200, 400 oder 600 mg Ibuprofen, insbesondere 684 mg Ibuprofen-Lysinat entsprechend 400 mg Ibuprofen.

Als mikrokristalline Cellulose wird vorzugsweise Sanaq® 102 oder Vivapur® 102, eine mikrokristalline Cellulose mit einem durchschnittlichen Polymerisationsgrad von 215-240 und einer Dichte (bulk) von 0,28 - 0,33 g/cm³, die z.B. von JRS - J. Rettenmaier USA LP, Schoolcraft, MI 49087 erhältlich ist, eingesetzt.

Als hochdisperses Siliziumdioxid wird vorzugsweise Aerosil® 200, eine hochreine kolloidale Kieselsäure, die z.B. von der Degussa AG, Weißfrauenstrasse 9, 60311 Frankfurt am Main erhältlich ist, verwendet.

Die Beschaffenheit des zu verwendenden Filmbildners ist an sich unkritisch. In der Regel wird eine wässrige Lösung eines Filmbildnersystems auf die Tablettenkerne aufgebracht. Bevorzugt wird einer der in der folgenden Tabelle 1 aufgeführten Materialien als Filmbildner eingesetzt:

**TABELLE 1**

| Markenname | Inhaltsstoffe | Hersteller |
|---|---|---|
| Opadry® I | HPMC, PEG & Pigment | Colorcon, West Point, PA |
| Opadry II ® | HPMC, PEG, Maltodextrin & Pigment | Colorcon, West Point, PA |
| Klucel® | Hydroxypropylcellulose | Hercules/Aqualon, Wilmington, DE |
| Natrosol® | Hydroxyethylcellulose | Hercules/Aqualon, Wilmington, DE |
| Kollidon® | Polyvinyl pyrrolidon | BASF, Parsippany, NJ |
| Kelton® | Natriumalginat | Kelco, San Diego, CA |
| Pharmazeutische Gelatine | Gelatine | Hormel Foods Corp., Austin, MN |

| | | |
|---|---|---|
| HPMC: Hydroxypropylmethylcellulose PEG: Polyethylenglykol Pigment: Titandioxid | | |

Eine weitere bevorzugte Ausführungsform der erfindungsgemäßen Filmtablette ist eine Ibuprofen-haltige Filmtablette, wobei der Tablettenfilm aus Opadry II® der Fa. Colorcon hergestellt wurde.

Besonders bevorzugt ist eine Ibuprofen-haltige Filmtablette, worin der Tablettenfilm 0,5 bis 5,0, insbesondere 2,0 bis 3,0 Gew.-% Opadry II® bezogen auf das Gesamtgewicht der Filmtablette enthält.

Die erfindungsgemäße Tablette kann durch direktes Mischen und Verpressen der Bestandteile oder durch Granulieren und Verpressen hergestellt werden.

Die Filmlösung wird durch Vermischen des Filmbildners mit Wasser hergestellt. Mit einer herkömmlichen Beschichtungspfanne kann diese Lösung auf die Tablettenkerne aufgebracht werden.

Die Presskräfte, die benötigt werden, Tabletten der geeigneten Bruchfestigkeiten und damit der gewünschten Zerfallszeiten herzustellen, sind von den Formen und Größen der verwendeten Stempelwerkzeugen abhängig. Vorzugsweise ist die Presskraft in einem Bereich von 2 - 20 kN. Höhere Presskräfte können zu Tabletten mit verlangsamter Wirkstoff-Freisetzung führen. Niedrigere Presskräfte können zu mechanisch instabilen Tabletten führen. Die Tablettenkerne können unterschiedliche Formate aufweisen, bevorzugt sind runde biplane oder bikonvexe und ovale oder oblong Formen.

Die erfindungsgemäßen Filmtabletten eignen sich zur Behandlung von akuten und chronischen Schmerzzuständen, insbesondere von Kopf-, Zahn- und Menstruationsschmerzen sowie bei Migräne. Dabei werden mit Hilfe der erfindungsgemäßen Filmtablette sehr schnell zur Schmerzbekämpfung ausreichende, hohe Blutplasma- und Wirkortkonzentrationen erreicht.

Das nachfolgende Beispiel dient der Illustration erfindungsgemäßer Formulierungen. Es ist lediglich als mögliche, exemplarisch dargestellte Vorgehensweise zu verstehen, ohne die Erfindung auf deren Inhalt zu beschränken.

### Beispiel 1

| Zusammensetzung | pro Tablette mg | Handelsname bzw. Lieferant |
|---|---|---|
| Ibuprofen-lysinat | 684,000 | |
| Mikrokristalline Cellulose 102 | 123,000 | Sanaq® 102 / Vivapur® 102 |
| Hochdisperses Siliciumdioxid | 3,000 | Aerosil® 200 |
| Magnesiumstearat | 10,000 | |
| Tablettenkern | 820,000 | |
| Opadry® white | 20,000 | Colorcon |
| + 6 % Fabrikationszuschlag | 1,200 | |
| Gereinigtes Wasser * | 152,000 | |
| | 840,000 | |

| | | |
|---|---|---|
| * im Fertigprodukt nicht mehr enthalten | | |

Die direkte Verpressung umfasst die Herstellung einer Mischung der Bestandteile des Tablettenkerns mit einem Mischer. Die Mischung wird gesiebt und in Tabletten mit abgerundeten Kanten gepresst.

Anschließend wird eine Lösung des Filmbildners in Wasser hergestellt, der auf die Tabletten aufgebracht wird.

Die so erhaltenen erfindungsgemäßen Tabletten zeigen in *in vitro* Untersuchungen zur Wirkstofffreisetzung ein mit Dolormin® vergleichbares bzw. teilweise geringfügig schnelleres und gleichmäßigeres Freisetzungsverhalten des Wirkstoffes.

## Patentansprüche

1. Ibuprofen-haltige Filmtablette **dadurch gekennzeichnet, dass** der Tablettenkern aus folgenden Komponenten besteht:
(a) 80,0-85,0 Gew.-% des Lysinats von racemischem (R/S)-Ibuprofen,
(b) 12,5-17,5 Gew.-% Mikrokristalline Cellulose,
(c) 0,1-1,0 Gew.-% hochdisperses SiO₂, und
(d) 0,75-1,5 Gew.-% Magnesium Stearat,
wobei sich die angegebenen Prozentangaben der jeweiligen Bestandteile auf das Gesamtgewicht der Filmtablette beziehen.

2. Ibuprofen-haltige Filmtablette nach Anspruch 1, wobei der Tablettenkern aus folgenden Komponenten besteht (%-Angaben beziehen sich auf Tablettenkern):
(a) 83,0-84,0 Gew.-% des Lysinats von racemischem (R/S)-Ibuprofen,
(b) 14,5-15,5 Gew.-% Mikrokristalline Cellulose,
(c) 0,3-0,4 Gew.-% hochdisperses SiO2, und
(d) 1,0-1,4 Gew.-% Magnesium Stearat.

3. Ibuprofen-haltige Filmtablette nach Anspruch 1 oder 2, **dadurch gekennzeichnet, dass** der Tablettenfilm aus Opadry II^{®} hergestellt wurde, wobei Opadry II^{®} aus Hydroxypropylmethylcellulose, Polyethylenglykol, Maltodextrin und Titandioxid besteht

4. Ibuprofen-haltige Filmtablette nach einem der Ansprüche 1 bis 3, **dadurch gekennzeichnet, dass** der Anteil des Tablettenfilms 0,5 bis 5,0 Gew.-% bezogen auf das Gesamtgewicht der Filmtablette beträgt.

5. Ibuprofen-haltige Filmtablette nach Anspruch 4, **dadurch gekennzeichnet, dass** der Anteil des Tablettenfilms 2,0 bis 3,0 Gew.-% bezogen auf das Gesamtgewicht der Filmtablette beträgt.

6. Verwendung einer Ibuprofen-haltigen Filmtablette nach einem der Ansprüche 1 bis 5 zur Herstellung eines Arzneimittels zur Behandlung von akuten und/oder chronischen Schmerzen.

7. Verwendung nach Anspruch 6 zur Herstellung eines Arzneimittels zur schnellen Erreichung einer zur Schmerzbekämpfung ausreichend hohen Blutplasma- und Wirkortkonzentration.

## Claims

1. Ibuprofen-containing film-coated tablet, **characterised in that** the tablet core consists of the following components:
(a) 80.0-85.0 wt.% of the lysinate of racemic (R/S)-ibuprofen,
(b) 12.5-17.5 wt.% of microcrystalline cellulose,
(c) 0.1-1.0 wt.% of highly dispersed SiO₂, and
(d) 0.75-1.5 wt.% of magnesium stearate,
the percentages given for the respective ingredients being based on the total weight of the film-coated tablet.

2. Ibuprofen-containing film-coated tablet according to claim 1, wherein the tablet core consists of the following components (percentages are based on the tablet core):
(a) 83.0-84.0 wt.% of the lysinate of racemic (R/S)-Ibuprofen,
(b) 14.5-15.5 wt.% of microcrystalline cellulose,
(c) 0.3-0.4 wt.% of highly dispersed SiO₂, and
(d) 1.0-1.4 wt.% of magnesium stearate.

3. Ibuprofen-containing film-coated tablet according to claim 1 or 2, **characterised in that** the tablet film has been produced from Opadry II^{®}, Opadry II^{®} consisting of hydroxypropylmethylcellulose, polyethyleneglycol, maltodextrin and titanium dioxide.

4. Ibuprofen-containing film-coated tablet according to one of claims 1 to 3, **characterised in that** the amount of the tablet film is 0.5 to 5.0 wt.% based on the total weight of the film-coated tablet.

5. Ibuprofen-containing film-coated tablet according to claim 4, **characterised in that** the amount of the tablet film is 2.0 to 3.0 wt.% based on the total weight of the film-coated tablet.

6. Use of an ibuprofen-containing film-coated tablet according to one of claims 1 to 5 for preparing a medicament for treating acute and/or chronic pain.

7. Use according to claim 6 for preparing a medicament for rapidly achieving a high enough blood plasma and activity site concentration to combat pain.

## Revendications

1. Comprimé pelliculé contenant de l'ibuprofène, **caractérisé en ce que** le noyau de comprimé consiste en les composants suivants :
(a) 80,0 à 85,0 % en poids de lysinate d'ibupro-fène racémique (R/S),
(b) 12,5 à 17,5 % en poids de cellulose microcristalline,
(c) 0,1 à 1,C % en poids de SiO₂ hautement dispersé et
(d) 0,75 à 1,5 % en poids de stéarate de magnésium,
dans lequel les pourcentages indiqués des composants respectifs se rapportent au poids total du comprimé pelliculé.

2. Comprimé pelliculé contenant de l'ibuprofène, selon la revendication 1, dans lequel le noyau de comprimé consiste en les composants suivants (les % se rapportent au noyau de comprimé) :
(a) 83,0 à 84,0 % en poids de lysinate d'ibupro-fène racémique (R/S),
(b) 14,5 à 15,5 % en poids de cellulose microcristalline,
(c) 0,3 à 0,4 % en poids de SiO₂ hautement dispersé et
(d) 1,0 à 1,4 % en poids de stéarate de magnésium.

3. Comprimé pelliculé contenant de l'ibuprofène selon la revendication 1 ou 2, **caractérisé en ce que** le film pelliculé a été produit à partir d'Opadry II^{®}, dans lequel l'Opadry II^{®} consiste en de l'hydroxypropylméthylcellulose, du polyéthylène glycol, de la maltodextrine et du dioxyde de titane.

4. Comprimé pelliculé contenant de l'ibuprofène selon l'une des revendications 1 à 3, **caractérisé en ce que** la proportion du film pelliculé est de 0,5 à 5,0 % en poids par rapport au poids total du comprimé pelliculé.

5. Comprimé pelliculé contenant de l'ibuprofène selon la revendication 4, **caractérisé en ce que** la proportion du film pelliculé est de 2,0 à 3,0 % en poids par rapport au poids total du comprimé pelliculé.

6. Utilisation d'un comprimé pelliculé contenant de l'ibuprofène selon l'une des revendications 1 à 5, pour la production d'un médicament pour le traitement des douleurs aiguës et/ou chroniques.

7. Utilisation selon la revendication 6, pour la production d'un médicament pour atteindre rapidement une concentration suffisamment élevée dans le plasma sanguin et le lieu d'action pour lutter contre la douleur.
